# EUROPÄISCHE PATENTSCHRIFT

(11) **EP 2 720 655 B1**
(45) Veröffentlichungstag und Bekanntmachung des Hinweises auf die Patenterteilung: **17.10.2018**
(21) Anmeldenummer: 12735105.4
(22) Anmeldetag: 18.06.2012
(51) Int. Cl.: A61F 5/56

(54) **INTRAORALES SCHNARCHTHERAPIEGERÄT**
INTRAORAL SNORING THERAPY DEVICE
DISPOSITIF INTRABUCCAL DE TRAITEMENT DU RONFLEMENT

(30) Priorität: 17.06.2011 EP 11170332
(43) Veröffentlichungstag der Anmeldung: 23.04.2014
(73) Patentinhaber: Dursteler, Carsten, 14109 Berlin (DE)
(72) Erfinder: Dursteler, Carsten, 14109 Berlin (DE)
(74) Vertreter: Hertin und Partner Rechts- und Patentanwälte PartG mbB
(86) Internationale Anmeldenummer: PCT/EP2012/061609
(87) Internationale Veröffentlichungsnummer: WO 2012/172112

(56) Entgegenhaltungen:
- EP-A1- 1 516 604
- WO-A1-2008/023799
- JP-A- 2004 073 473
- US-A- 5 947 724
- US-A1- 2004 177 853
- US-A1- 2007 183 572

## Beschreibung

Die Erfindung betrifft ein intraorales Schnarchtherapiegerät und ein Verfahren zu dessen Herstellung umfassend analoge und/oder digitale Abformung eines Unter- und Oberkiefers, Datenaufbereitung der abgeformten Kiefer, Modulation des Schutzes und Fertigung des Schnarchtherapiegerätes

Schnarchen ist ein weit verbreitetes Problem. Etwa 40 % der Bevölkerung der Industriestaaten schnarcht. Bei älteren Menschen ist dieser Prozentsatz sogar noch größer, da die muskuläre Straffung der pharyngealen Atemwege, also der Gewebeteile des Rachenraums, mit fortschreitendem Alter abnimmt. Im Schlaf entspannen sich die pharyngealen Gewebeteile und verengen den freien Querschnitt, der für den Luftaustausch beim Ein- und Ausatmen zur Verfügung steht. In Folge dessen erhöht sich die Strömungsgeschwindigkeit der Atemluft und die entspannten Gewebeteile der pharyngealen Atemwege werden zu flatternden Bewegungen angeregt. Die mit diesen flatternden Bewegungen einhergehende Geräuschbildung wird als Schnarchen bezeichnet.

Die Verengung der pharyngealen Atemwege während des Schlafens kann so ausgeprägt sein, daß es zum Verschluß des Atemwege und damit zu einem Atemstillstand kommt. Bei der sogenannten Schlafapnoe handelt es sich um eine ernstzunehmende Krankheit, da der Atemstillstand durchaus mehrere Sekunden andauern kann. Anschließend setzt die Atmung wieder explosionsartig und in der Regel geräuschvoll ein. Schlafapnoe führt in der Regel zu Tagesmüdigkeit und ernsten Gefäßkrankheiten. Auch normales Schnarchen ohne Atemstillstand kann Tagesmüdigkeit und Unkonzentriertheit hervorrufen.

Die Folgen des Schnarcher betreffen nicht nur die Schnarcher selbst, sondern oft auch deren Partner, bei denen häufig vergleichbare Symptome festgestellt werden. Damit ist der Anteil der Bevölkerung, der von den Folgen des Schnarchens betroffen ist, noch weitaus größer als der ohnehin schon hohe Anteil der Schnarcher.

Zur Verhinderung des Schnarchens sind eine ganze Reihe von Therapiegeräten bekannt geworden. Neben extraoral angewendeten Vorrichtungen, welche vom Schnarcher als sehr lästig empfunden werden, sind weiterhin intraorale Therapiegeräte bekannt, die in der Regel eine Verengung des pharyngealen Anteils der Atemwege während des Schlafs verhindern sollen.

Bekannte, intraorale Schnarch-Therapiegeräte enthalten beispielsweise Bügel und plattenförmige Mittel zum Niederhalten der Zunge oder eingelagerte Magnete, welche durch gegenseitige Abstoßung die Kiefer in einem vorbestimmten Abstand halten sollen. Bei anderen Therapiegeräten wird die Zunge angesaugt, um während des Schlafs ein Zurückrutschen der Zunge in den Rachenraum zu verhindern. Das Funktionsprinzip wieder anderer Therapiegeräte beruht darauf den Schnarcher zur Nasenatmung zu zwingen. Dadurch wird die Intensität des Schnarchens verringert.

Weiterhin offenbart die WO 2008/023799 beschreibt ein Schnarchtherapiegerät, welches eine Oberkieferschiene und Unterkieferschiene aufweist. Die Schienen sind mit zwei seitlich angebrachten Bändern verbunden und die Bänder können aus einem flexiblen Material gefertigt sein. Auch US2004177853 offenbart Schienen die mit zwei seitlich angebrachten Bändern verbunden sind. US5947724 offenbart Schienen die mit zwei angebrachten Bändern mit eine Shore-Härte von 60-80 verbunden sind.

Die Druckschriften EP 1516604, DE 201007007760 und DE 20102432 zeigen Schnarchtherapiegeräte, die zwei Schienen umfassen. Auch aus der JP 2004073473 ist ebenfalls ein Schnarchtherapiegerät bekannt.

Aus der Praxis ist weiterhin ein Schnarchtherapiegerätder eingangs genannten Art bekannt, das im Vergleich zu den übrigen, bekannten Vorrichtungen einen verbesserten Tragekomfort bietet. Dieses Schnarchtherapiegerätweist je eine Schiene für den Ober- und Unterkiefer auf, die durch zwei Führungsstäbe verbunden sind. Die beiden Stäbe sind dabei mit ihren Enden jeweils so an den Außenseiten einer Ober- und einer Unterkieferschiene gelenkig angeordnet, daß Ober- und Unterkieferschiene gegeneinander verschwenkbar sind und daß der Unterkiefer des Trägers nach vorne bewegt wird, wenn er beim Einschlafen nach unten absinkt. Diese anteriore bzw. ventrale Verschiebung des Unterkiefers bewirkt eine Öffnung der pharyngealen Atemwege.

Nachteilig bei den bekannten Schnarchtherapiegeräten ist, daß der Träger die seitlich angeordneten Stäbe als störend empfindet. So kann es an den Wangeninnenseiten im Bereich der Stäbe, insbesondere im Bereich der Anlenkpunkte der Stäbe, zu unangenehmen Druckstellen kommen. Durch eine vermehrte Bewegung des Unterkiefers während des Tragens des Schnarch-Therapiegerätes kann es zudem zu Reizungen, im schlimmsten Fall zu Entzündungen des Gewebes an der Innenseite der Wangen kommen. Darüber hinaus kommt es zu einer steten Berührung zwischen Wangen und Stäben, wenn das Gerät getragen wird. Aus diesem Grunde hat der Schnarcher stets das unangenehme Gefühl, als ob er einen Fremdkörper im Mund tragen würde. Weiterhin ist die Bewegungsmöglichkeit des Unterkiefers stark eingeschränkt, was zu Verspannungen der Muskulatur führen kann. Wegen dieser Nachteile kann es, wie bei den anderen bekannten Vorrichtungen auch, zur Nichtbenutzung dieser Schnarch-Therapiegeräts kommen. Außerdem müssen die bekannten Schnarchtherapiegeräte häufig gereinigt, bzw. desinfiziert werden, da sich zahlreiche Mikroorganismen oder Verunreinigungen auf der Oberfläche festsetzen. Ein weiteres Problem des Standes der Technik ist, dass ein Abrutschen der Zunge nicht vollständig ausgeschlossen werden kann.

Im Stand der Technik sind verschiedene Techniken zur Herstellung eines Mundeinsatzes beschrieben. Hierfür nimmt beispielsweise ein Zahnarzt einen Abdruck der Mundhöhle eines Patienten, um hieraus einen Gipsguß herzustellen, der nachfolgend als Modell genutzt wird. In den Gipsguß wird beispielsweise ein Acylharzmaterial eingefüllt und zur Polymerisation gebracht, wodurch ein bearbeitbarer Abdruck der Mundhöhle entsteht. Das polymerisierte Material kann durch einen Techniker bearbeitet werden, um eine optimale Passung zu garantieren. Der Einsatz muss mit den Zähnen abschließen, was nur durch mehrmaliges Testen des Einsatzes durch den Patienten erreicht werden kann. Es ist ein mühsames und zeitaufwendiges Verfahren, welches sich für den Patienten nicht als angenehm gestaltet und häufig keine optimale Passung darstellt. Außerdem sind die Herstellungskosten unausweichlich groß.

Im Stand der Technik sind weiterhin Mundeinsätze beschrieben, welche direkt im Mund entweder vom Zahnarzt oder vom Benutzer selbst angepasst werden. Solche Mundeinsätze bestehen in der Regel aus thermoplastisch verformbarem Kunststoff, welcher nach dem Erweichen im Wasserbad (Boil and bite) oder mit Hilfe einer anderen Wärmequelle, wie z. B. einem Heißluftgebläse oder einem Infrarotstrahler, mittels Finger, Zunge und perioraler Muskulatur direkt im Munde angepasst wird. Diese Mundeinsätze werden grob geformt und konfektioniert in den Handel gebracht und vielfach verwendet.

Im Stand der Technik ist offenbart, dass Dentalschienen oder Mundeinsätze mittels einer Tiefziehtechnik hergestellt werden können, bei der thermoplastische Folien in Tiefziehgeräten erwärmt und über ein Kiefermodell beispielsweise aus Gips verformt werden. Tiefziehfolien werden in den verschiedensten Formen und Größen sowie in unterschiedlichen Farben angeboten. Die Folienform richtet sich nach dem für die Verarbeitung empfohlenen Tiefziehgerät, die Dicke der Folie nach der angestrebten Konstruktionsform und der Anwendung des Schnarchtherapiegerätes. Die Folien können beispielsweise aus Ethylen-Vinylacetat-Copolymer bestehen. Aus den Folien kann die gewünschte Verarbeitungsform herausgestanzt werden, welche im Labor durch Erwärmung und mittels Druck in die gewünschte Form bzw. auf das jeweilige Modell aufgebracht wird. Verschiedene Tiefziehgeräte finden dabei Anwendung, deren Unterschiede unter anderem in der Erwärmungs- und der Tiefziehtechnik liegen. Unterschieden wird dabei zwischen Vakuum- und Druckformtechnik. Um eine notwenige Stabilität und Stärke des Schnarchtherapiegerätes zu garantieren, werden mehrere Folienschichten übereinander aufgezogen, was nicht nur sehr kostenaufwendig, sondern auch arbeits- und zeitaufwendig ist.

Ausgehend von diesem Stand der Technik ist es die Aufgabe der vorliegenden Erfindung eine Schnarchtherapiegerät bereitzustellen, das passgenau für einen Patienten anfertigbar ist und nicht die Nachteile oder Mängel des Stands der Technik aufweist.

Gelöst wird die Aufgabe durch die unabhängigen Ansprüche. Vorteilhafte Ausführungsformen ergeben sich aus den Unteransprüchen.

Es wird eine intraorales Schnarchtherapiegerät mit einer Oberkieferschiene und einer Unterkieferschiene bereitgestellt, wobei die Oberkieferschiene und die Unterkieferschiene über mindestens zwei Zugbänder miteinander verbunden sind und die Zugbänder mit ihrem ersten Ende an der Oberkieferschiene und ihrem zweiten Ende an der Unterkieferschiene befestigt sind, so daß die Oberkieferschiene und die Unterkieferschiene relativ zueinander bewegbar sind. Es kann auch bevorzugt sein lediglich ein Zugband oder mehr als zwei Zugbänder zu verwenden.

Durch die Erfindung ergeben sich eine Reihe von Vorteilen. Durch die Anordnung der Zugbänder und deren bevorzugt eleastische Eigenschaften weist das erfindungsgemäße Therapiegerät keine von der Oberkieferschiene und der Unterkieferschiene seitlich abstehenden, kantigen und/oder beweglichen Bauteile auf, die beim Tragen des Schnarchtherapiegerätes, also imGebrauchszustand, in direkten und dauerhaften Kontakt mit den Wangen kommen. Probleme wie beim Stand der Technik, insbesondere Reizungen des Gewebes an der Innenseite der Wangen, können nicht auftreten. Da die beim Stand der Technik als störend empfundenen Stäbe durch die Verwendung eines vorzugsweise eleastischen Bandes im Bereich der Aufbißflächen quasi verschwinden, erhöht sich der Tragekomfort des erfindungsgemäßen Therapiegerätes. Gleichzeitig wird die Akzeptanz beim Benutzer und damit die Tragehäufigkeit verbessert. Es ist bevorzugt, dass die Oberkiefer- und Unterkieferschiene spezielle Ebenen aufweist, die ein ungehindertes Gleiten der Kiefer zueinander ermöglichen. Die Ebenen sind vorzugsweise parallel zur Kauebene des Patienten ausgerichtet. Weiterhin ist bevorzugt, dass im Bereich der Molaren Erweiterungen in Form einer Stufe vorliegen, die eine Rückverlagerung des Unterkiefers verhindert.

Erschlafft beim Einschlafen die Unterkiefermuskulatur, sinkt der Unterkiefer nach unten. Im Gebrauchszustand, also im in den Mund eingesetzten Zustand, bewirkt das Schnarchtherapiegerät eine zunehmende Verschiebung des Unterkiefers insbesondere nach vorne (anterior). Die Verschiebung des Unterkiefers im Gebrauchszustand wird über die Anordnung der Zugbänder an der Ober- und Unterkieferschiene bestimmt. Durch die anteriore Verschiebung des Unterkiefers wird eine Öffnung der pharyngealen Atemwege erreicht, so daß die Atemluft ungestört in die Luftröhre eindringen und aus dieser entweichen kann, ohne erschlafftes Gewebe in flatternde Bewegung zu versetzen. Die Schienen des erfindungsgemäßen Schnarchtherapiegerät sind so angeordnet, dass der Unterkiefer nach vorne verlagert wird und so ein Erschlaffen des Unterkiefers, bzw. Abrutschen der Zunge verhindert werden kann. Nichts dergleichen findet sich im Stand der Technik. Diese überraschenden Vorteile in Kombination mit dem erheblich verbesserten Tragekomfort, grenzen das Schnarchtherapie deutlich vom Stand der Technik ab.

Eine Ausnehmung zur Aufnahme der Zugbänder in der Oberkieferschiene und der Unterkieferschiene, das heißt im Bereich der seitlichen Aufbißflächen ist vorgesehen. Die Ausnehmung sollte derart ausgebildet sein, daß die Zugbänder in geschlossenem Zustand des Therapiegerätes derart darin aufgenommen ist, daß die Oberkieferschiene und die Unterkieferschiene im Bereich der einander zugewandten Aufbisse zumindest teilweise aufeinander aufliegen, und zwar derart, daß jegliche Störung der Okklusion durch die Bänder ausgeschlossen ist. Im Ergebnis ergibt sich damit eine Einbettung der Bänder in den seitlichen, einander zugewandten Aufbissen der Schienen. Im geschlossenen Gebrauchszustand des erfindungsgemäßen Schnarchtherapiegerätes ist damit jede Beeinträchtigung und Einschränkung der Zunge und der Wangenmuskulatur ausgeschlossen. Um eine genau vorgegebene Verstellung des Unterkiefers in Abhängigkeit des Öffnungswinkels zwischen Ober- und Unterkieferschiene im Gebrauchszustand zu erzielen, sollten die Zugbänder in Längsrichtung weder dehnbar noch ausziehbar sein. Bevorzugt werden die Bänder aus flexiblem Kunststoff verwendet, um eine; freie Beweglichkeit des Unterkiefers allerdings mit Ausnahme der Längsrichtung zu ermöglichen. Weiter bevorzugt sind solche flexiblen Kunststoffe, die eine Teil der Bewegungsenergie des Unterkiefers im Gebrauchszustand aufnehmen und dadurch die durch die Bewegung des Unterkiefers auf die Befestigungspunkte der Bänder einwirkenden Kräfte verringern. Auf diese Weise kann das Schnarchtherapiegerät trotz hoher mechanischer Belastung während des Tragens vergleichsweise einfach und klein bauend ausgeführt werden.

Liegen Ober- und Unterkieferschiene des erfindungsgemäßen Geräts im Bereich der seitlichen Aufbisse aufeinander auf (geschlossener Zustand), befinden sich die Bänder in einem spitzen Winkel zur seitlichen Aufbißebene. Bei konsanter Länge der Bänder und konstantem Öffnungswinkel zwischen Ober- und Unterkieferschiene ist die durch das erfindungsgemäße Schnarchtherapiegerät im Gebrauchszustand hervorgerufene Längsverschiebung des Unterkiefers um so größer, je kleiner der Winkel zwischen Bänder und Aufbißebene ist. Dadurch können die pharyngealen Atemwege bereits durch einen geringen Öffnungswinkel zwischen Ober- und Unterkiefer geöffnet werden.

Das erfindungsgemäße Schnarchtherapiegerät erlaubt es in einfacher Weise, unterschiedliche Längen der Bänder vorzusehen, um das Gerät an die anatomischen Anforderungen des Träger anzupassen.

Die Zugbänder sind derart an der Oberkieferschiene und an der Unterkieferschiene befestigt, daß der Unterkiefer im Gebrauchszustand bei in den Mund eingesetztem Therapiegerät mit dem Öffnen des Mundes nach vorne bewegt wird.

Die Erfindung betrifft weitehrin ein Verfahren zur Herstellung des Schnarchtherapiegerätes, umfassend die folgenden Schritte:
a. analoge und/oder digitale Abformung mindestens eines Kiefers oder eines Zahnes,
b. Datenaufbereitung der unter a. bereitgestellten Abformung, d. h. der abgeformten Kiefer und/oder Zähne,
c. digitale Modulation des Schnarchtherapiegerätes und
d. Fertigung des Schnarchtherapiegerätes.

Es war völlig überraschend, dass ein bekanntes Verfahren zur Herstellung eines Schnarchtherapiegerätes verwendet werden kann, wobei das Schnarchtherapiegerät bevorzugt in Übereinstimmung mit einem Zahnbogen ausgeformt ist und vorteilhafterweise passend die Flächen und Seiten der Zähne abdeckt.

Ein nicht beanspruchtes Beispiel betrifft die Verwendung eines Verfahrens zur Herstellung eines Schnarchtherapiegerätes. Hierdurch wird ein hoher Tragekomfort erreicht, ein optimaler Schutz garantiert und überraschenderweise nicht die Atmung beeinflusst. Es hat sich weiterhin gezeigt, dass das Schnarchtherapiegerät leicht zu reinigen ist und bakterizid wirkt, so dass ein dauerhafter Einsatz sichergestellt wird. Ein weiterer Vorteil des bevorzugten Verfahrens ist, dass das Schnarchtherapiegerät zwar aus einzelnen separaten Schichten besteht, aber als eine untrennbar miteinander verbundene Einheit vorliegt. Hierdurch kann eine hohe Stabilität des Schnarchtherapiegerätes garantiert werden. Dies ist ein wesentlicher Vorteil gegenüber dem Folientiefziehverfahren, bei dem die Schichten zwar thermoplastisch miteinander verbunden werden, aber noch als getrennt aufgetragene Schichten vorliegen. Das Folientiefziehverfahren ist ein aufwendiges Verfahren, welches zahlreiche Arbeitsschritte benötigt. Zudem betrifft das nicht beanspruchte Beispiel auch ein Schnarchtherapiegerät hergestellt durch ein Verfahren umfassend die folgenden Schritte:
a. analoge und/oder digitale Abformung mindestens eines Kiefers oder eines Zahnes,
b. Datenaufbereitung der unter a. bereitgestellten Abformung, d. h. der abgeformten Kiefer und/oder Zähne,
c. digitale Modulation des Schnarchtherapiegerätes und
d. Fertigung des Schnarchtherapiegerätes.

Durch das Verfahren kann ein Schnarchtherapiegerät in wenigen Arbeitsschritten hergestellt werden, wobei die Modellierung digital mit einem CAD-System und die Fertigung mittels einem CAM-System erfolgt, wodurch Arbeitszeit und Arbeitskraft eingespart werden können. Insbesondere durch die digitale Modellierung kann das Schnarchtherapiegerät optimal an einen Kiefer angepasst werden und aus unterschiedlichen Materialien aufgebaut sein, die vorzugsweise untrennbar miteinander verbunden sind. Auch können vorteilhafterweise Bereiche des Schnarchtherapiegerät unterschiedlich gestaltet werden, indem beispielsweise in unterschiedlichen Schichten unterschiedliche Materialien verwendet werden. So kann es beispielsweise vorteilhaft sein, wenn die Zugbänder aus einem anderen Material wie die Ober- und Unterkieferschiene gefertigt sind. Es kann jedoch auch bevorzugt sein, dass die Schienen und/oder die Zugbänder aus dem gleichen Material sind. Weiterhin kann es vorteilhaft sein, wenn die Schienen nicht aus mehreren Schichten aufgebaut sind.

Durch die bevorzugte Verwendung des Verfahrens, kann ein Schnarchtherapiegerät bereitgestellt werden, das eine optimale Passung aufweist und schnell und kostengünstig herstellbar ist. Hierfür wird von einer Mundhöhle ein Abdruck gemacht. Der Abdruck oder die Abformung kann mittels analoger und/oder digitaler Verfahren erfolgen, wobei mindestens ein Kiefer und/oder mindestens ein Zahn erfasst wird. Die analoge Abformung umfasst bevorzugt Abdruckverfahren, die im Sinne der Erfindung insbesondere als herkömmliche Abdruckverfahren bezeichnet werden. Es wird bei den herkömmlichen Abdruckverfahren ein Abdruck mittels einem verformbaren und aushärtbaren Material gemacht, wobei dieser mit beispielsweise Gips ausgegossen wird. Als Material kann bevorzugt Alginat (Salze der Alginsäure), Silikon, Hydrokolloide oder Polyäther eingesetzt werden. Das so erstellte Modell kann in nachfolgenden Verfahrensschritten bearbeitet werden.

Als Abformmaterial ist Alginat physiologisch unbedenklich und gestattet Abformungen von akzeptabler Genauigkeit und Detailtreue. Bei nicht sachgemäßer Weiterverarbeitung trocknet die Alginatabformung aus und kann dadurch unbrauchbar werden. Das Abformmaterial kann vorteilhafterweise in geeigneten Trägern aus Metall oder Kunststoff in den Mund eingebracht werden (sogenannte Abformlöffel).

Polyäther ist insbesondere eine übergreifende Bezeichnung für Polymere, deren organische Wiederholungseinheiten Ether-Funktionalitäten (C-O-C) in der Hauptkette enthalten. Es können eine Vielzahl strukturell sehr unterschiedlicher Polymere als Polyäther bezeichnet werden, umfassend. die Polyalkylenglycole (Polyethylenglycole, Polypropylenglycole und Polyepichlorhydrine) als Polymere von 1,2-Epoxiden, Polytetrahydrofurane (Polytetramethylenglycole), Oxetan-Polymere, Polyarylether oder Polyetheretherketone. Polyäther ist initial hydrophil, kann aber hoch präzise und auch unter schweren Bedingungen sehr passgenau in der Mundhöhle eingepasst werden. Es ist vorteilhafterweise geruchs- und geschmacksneutral und leicht aus dem Patientenmund zu entnehmen.

Weiterhin kann Silikon als Abformmaterial verwendet werden. Silikone umfassen im Sinne der Erfindung synthetische polymere Verbindungen, in denen Silicium-Atome über Sauerstoff-Atome kettenartig und/oder netzartig verknüpft sind, wobei die restlichen Valenzen des Siliciums durch Kohlenwasserstoff-Reste (z. B. Methyl-Gruppen, Ethyl-Gruppen, Propyl-Gruppen, Phenyl-Gruppen u. a.) abgesättigt sind. Silikonabdrücke sind hydrophop und aufgrund der mechanischen Eigenschaften unempfindlich und formstabil.

Es kann auch bevorzugt sein, Hydrokolloide als Abformmaterial zu verwenden. Hydrokolloide bezeichnen insbesondere (teilweise) wasserlösliche natürliche oder synthetische Polymere, die in wässrigen Systemen Gele oder viskose Lösungen bilden. Bevorzugte Hydrokolloide umfassen Carrageen, Pektine, Tragant Celluloseether, und Dextran. Hydrokolloidabdrücke sind sehr empfindlich auf Druckeinwirkung und Austrocknung, wobei sich hoch präzise Abdrücke anfertigen lassen.

Die so bereitgestellten Abdrücke der Zähne und/oder Kiefer werden bevorzugt mit einer Gipsmasse ausgegossen, damit eine stabile, beständige und bearbeitbare Form des Abdrucks entsteht.

Die Abformung kann weiterhin mittels einem digitalen Abformungsverfahren erfolgen. Bevorzugt wird die digitale Abformung optisch, insbesondere mittels Lava Chairside CO/S, Hint-Els Direkt Scan oder Cerec Connect Bleucam oder ähnlichem durchgeführt. Hierbei kann mit einem Handstück berührungslos über die Zähne geglitten werden und so eine präzise Abbildung der Mundsituation erzielt werden. Die gewonnenen Daten werden dann beispielsweise an einen Fachmann, z. B. einen Zahntechniker weitergeleitet.

In einer bevorzugten Ausführungsform kann das Handstück, welches in den Mundraum des Patienten eingeführt wird, insgesamt 22 Kameralinsen, 192 blaue LEDs und drei CCD-Sensoren beinhalten. So kann in kürzester Zeit eine hohe Datendichte erfasst werden. Die Daten werden bevorzugt in Echtzeit verarbeitet, so dass das Bild des Mundraums während des Scannens zeitgleich auf einem Bildschirm dargestellt wird. Es können alle Oberflächen trotz extremer Geschwindigkeit gleichmäßig eingescannt werden. Durch die digitale Abformung können hohe Tiefenschärfe, erhöhte Abbildungstreue am Präparationsrand und eine automatische Bildauslösung für verwacklungsfreie Aufnahmen erreicht werden.

Im Falle der analogen Abformung, erfolgt nach der Fertigstellung des Modells eine Datenaufbereitung, bei der das Modell (die abgeformten Kiefer und/oder Zähne) eingescannt, digitalisiert, gespeichert und/oder die Datensätze umgewandelt werden. Bei den analogen Abdruckverfahren liegt bevorzugt ein Gipsabdruck vor, der vorteilhafterweise eingescannt und digitalisiert wird. Hierfür können dem Fachmann bekannte Datenverarbeitungsprogramme genutzt werden, umfassend Lava Scan ST/3M Espe, D250-D640-D700/3Shape, Ceramill Map 100-300/Amann Girbach, Scan 200 /Bien air Cercon Eye/Degudent GmbH, Opernscan 100/Laserdenta, Sntech 3D/Smartoptics, Etkon es1/Straumann GmbH AnyScan/Reitel GmbH, NobelProcera/Nobelbiocare GmbH, Freie Form Software/SensAble oder DentalDesigner/3Shape.

In einer bevorzugten Ausführungsform scannt ein berührungsloser, optischer 3D-Scanner das Modell, z. B. den Gipsabdruck, wobei das Arbeitsprinzip des Scanners beispielsweise auf Streifenprojektion in Verbindung mit Triangulationsmethoden basieren kann. Hierbei wird ein Streifenmuster auf die Oberfläche des Modells projiziert und von einer Videokamera aus einem bestimmten Winkel aufgenommen werden. Um die gesamte Oberfläche darzustellen, werden weitere Ansichten hinzugefügt. So kann eine hohe Datendichte und große Genauigkeit erreicht werden. Es ist bevorzugt, dass der Scanner ein Bestandteil eines CAD/CAM-Systems ist, das insbesondere aus einem Scanner, einer Fräseinheit, einem Sinterofen und den entsprechenden Materialien Zirkonoxid und Verblendkeramik bestehen kann.

Der Abdruck wird vorteilhafterweise eingescannt und digitalisiert. Die bereits digital vorliegenden Abformungen und/oder die digitalisierten Abdrücke werden bevorzugt in ein Dateiformat umgewandet, welches von einem Datenverarbeitungsprogramm, insbesondere einem Modulationsprogramm gelesen und bearbeitet werden kann. Ein bevorzugtes Datenformat ist STL, IGES, STEP, SLC, OBJ, DF oder Parasolid. Die digitale Modulation und/oder Herstellung des Schnarchtherapiegerätes kann vorteilhafterweise mittels einem Datenverarbeitungsprogramm, bevorzugt CAD/CAM-System erfolgen. Bevorzugt ist ein 3D-Modelliersystem Bestandteil des CAD/CAM-Systems. Die Daten können vorteilhafterweise zur weiteren Verarbeitung an bestehende CAD/CAM-Systeme exportiert werden. Durch das System können feinste Oberflächenstrukturen generiert werden, die bevorzugt über hochauflösende Rapid-Prototyping Verfahren, HSC-Frästechnologien und Lasergravurmaschinen umgesetzt werden können. Ein CAD/CAM-System (CAD = "computer aided design" und CAM = "computer aided manufacturing") bietet eine optimale Verknüpfung von einem Modellierprogramm und einem Fertigungsprogramm. CAD bezeichnet im Sinne der Erfindung insbesondere die Verwendung eines Computers als Hilfsmittel beim technischen Zeichnen. Die Zeichnung wird bevorzugt als dreidimensionales Objekt (3D) mit Hilfe des Computers angefertigt. Hierbei kann das Schnarchtherapiegerät auf einem virtuellen Model bevorzugt in mehreren Lagen, insbesondere Polymerlagen dargestellt und konstruiert werden. Vorteilhafterweise besteht das Schnarchtherapiegerät nicht aus mehreren voneinander getrennten Lagen, sondern die Lagen bilden eine homogene Schicht. Die Lagen sind untrennbar miteinander verbunden, wobei die Schichten auch ineinander verlaufen können. Dies ist ein wesentlicher Vorteil gegenüber dem Folientiefziehverfahren, da bei diesem die Schichten separat aufgebracht werden. Bei dem erfindungsgemäßen Verfahren verschmelzen die Schichten homogen miteinander, wodurch eine hohe Stabilität garantiert werden kann. Außerdem ist das Folientiefziehverfahren sehr arbeits- und zeitaufwendig, da die Schichten nacheinander auf ein Modell aufgebracht werden müssen. Die erfindungsgemäße Verwendung des Verfahrens hingegen kann schnell und einfach durchgeführt werden.

Das auf die Zahnreihe aufgebrachte Polymer oder die Schiene weist eine Dicke von 3 bis 6 mm auf. Es hat sich herausgestellt, dass die bevorzugte Dicke des Schnarchtherapiegerätes einen optimalen Schutz darstellt und auch überraschenderweise ein Verrutschen des Unterkiefers effektiv verhindert. Es ist weiterhin bevorzugt, dass die Lagen in unterschiedlicher Stärke (oder Dicke) vorliegen und einen unterschiedlichen Härtegrad haben. Lagen, die zur Mundhöhle hin orientiert sind, weisen bevorzugt einen höheren Härtegrad als Lagen auf, die zum Zahn hin orientiert sind. Vorteilhafterweise kann der Härtegrad laufend ansteigen und muss nicht in separate Schichten unterteilt sein, wobei die Schichten verlaufend in das Schnarchtherapiegerät eingebracht sind. Das heißt, der Härtegrad kann vorteilhafterweise, bevorzugt auch in einer Schicht, variieren, so dass eine optimale Passung gewährt sind. Die Schichten weisen vorzugsweise keine starre Begrenzung auf, sondern können bevorzugt ineinander verlaufen, so dass eine optimale Verbindung der Schichten sichergestellt ist.

Es war völlig überraschend, dass der Härtegrad nicht auf lokale Stellen begrenzt sein muss, sondern optimal verteilt in dem Schnarchtherapiegerät vorliegen kann. Demgemäß besteht das Schnarchtherapiegerät zwar in einer bevorzugten Ausführungsform aus separaten Schichten, die unterschiedliche Härtegrade aufweisen, aber die Schichten untrennbar miteinander verbunden sind. Es ist bevorzugt dass die Shore-Härte einer Schicht im Bereich von 27 bis 95 liegt. Die Zugbänder haben eine Shore-Härte von 20 bis 70. Die Shore-Härte ist im Sinne der Erfindung ein Werkstoffkennwert, der insbesondere den Härtegrad von Kunststoffen und Elastomeren kennzeichnet. Bevorzugte Materialien umfassen beispielsweise VeroWhite, TangoBlack, TangoGray, TangoPlus, TangoBlackPlus oder DurusWhite. Vorteilhafterweise kann in einer bevorzugten Ausführungsform ein Sport Schnarchtherapiegerät bereitgestellt werden, bei dem fließende Übergänge zwischen unterschiedlichen Härtegraden bestehen. Es kann auch bevorzugt sein, dass eine Schicht unterschiedliche Härtegrade aufweist. Bei einem CAM-System werden Rohdaten für die Fertigung gesammelt, in Arbeitsschritte sortiert und mit Berücksichtigung von Abhängigkeiten verwaltet. Dazu gehören Schnittstellen, die z. B. Maße und Materialien wie auch Stücklisten aus CAD-Systemen einlesen und zur Bearbeitung bereitstellen. Zusätzlich zu den bereits in Rechnern enthaltenen Merkmalen werden ergänzende Informationen eingepflegt. Das können z. B. der Abgleich von Zeichnungen aus CAD und Stücklisten aus der Produktionsplanung und -steuerung sein, aber auch rein für die Fertigung erforderliche Daten wie Werkzeugbedarf, Spannpläne und Reihenfolge zur Bearbeitung. Im System werden dann gezielt die direkt von den Maschinen zu bearbeitenden Codes oder Materialanforderungen bereitgestellt und jeweils vom Bediener abgerufen. Auch in diesem Bereich werden Schnittstellen z. B. zur Maschinensteuerung oder einem Lagersystem benötigt. Nach erfolgter Bearbeitung bzw. dem Abschluss der Fertigungsschritte sind die verifizierten Ergebnisse eventuell weiter aufzubereiten und über weitere Schnittstellen an andere Prozesse in der computerintegrierten Produktion zu übermitteln. Das CAM-System umfasst bevorzugt einen 3D-Drucker. Das heißt, es ist bevorzugt, dass das Schnarchtherapiegerät mit einem 3D-Drucker, insbesondere einem Multilayerdrucker speziell gefertigt wird. Bevorzugte Drucker umfassen Objet3D Drucker oder Drucker der Connex-Familie oder Drucker der Firma envisionTEC. Insbesondere bevorzugt ist ein Perfactory 4 Standard mit ERM der Firma envisionTEC, insbesondere der Perfactory 4 Digitale Dental Drucker mit ERM. Der bevorzugte Drucker besitzt eine Auflösung von 1920x1200 Pixeln und eine native Pixelgröße von 0,0024" (60µm), eine Pixelgröße mit ERM von 0,0012" (30µm) und eine dynamische Voxel Auflösung in Z (materialabhängig) von 0,0098" (25µm) bis 0,0040" (100µm). Dem Fachmann ist bekannt, dass der Begriff Plotter synonym für 3D-Drucker verwendet werden kann. Mit den bevorzugten 3D-Druckern kann einfach und schnell ein Objekt, beispielsweise ein Schnarchtherapiegerät aus einem oder mehreren unterschiedlichen Materialien gefertigt werden. Es war völlig überraschend, dass die Drucker auch zur Herstellung eines Schnarchtherapiegerätes verwendet werden können. In einer bevorzugten Ausführungsform der Erfindung werden Ober- und Unterkieferschiene in einem Vorgang geplottet. Das Zugband kann entweder direkt mit den Schienen geplottet werden, oder separat. Nichts dergleichen ist aus dem Stand der Technik bekannt.

Nach einer vorteilhaften Weiterbildung der Erfindung ist das Schnarchtherapiegerät aus einem Kunststoffmaterial hergestellt. Die besonders einfache Verarbeitbarkeit eines Kunststoffmaterials erleichtert die Herstellung eines Schnarchtherapiegerätes. Zudem kann bei der Wahl eines zumindest teilweise elastischen Kunststoffs bereits eine durch die Materialeigenschaften hervorgerufene Dämpfungswirkung erzielt werden. Kunststoffe bezeichnen insbesondere Materialien, deren wesentliche Bestandteile aus solchen makromolekularen organischen Verb. bestehen, die synthetisch oder durch Abwandeln von Naturprodukten entstehen. Sie sind in vielen Fällen unter bestimmten Bedingungen (Wärme u. Druck) schmelz- und formbar. Zu den Kunststoffen gehören auch die Kautschuke und die Chemiefasern. Für die vorteilhafte Ausführungsform können Kunststoffe aus der Gruppe abgewandelte Naturstoffe, synthetische Kunststoffe (Polykondensate, Polymerisate, Polyaddukte), Duroplaste, und/oder ungesättigte Polyesterharze, umfassend Cellulosenitrat, Celluloseacetat, Cellulosemischester, Celluloseether, Polyamid, Polycarbonat, Polyester, Polyphenylenoxid, Polysulfon, Polyvinylacetal, Polyethylen, Polypropylen, Poly-1-buten, Poly-4-methyl-1-penten, lonomere, Polyvinylchlorid, Polyvinylidenchlorid, Polymethyl-methacrylat, Polyacrylnitril, Polystyrol, Polyacetal, Fluor-Kunststoffe, Polyvinylalkohol, Polyvinylacetat, Poly-p-xylylen, lineare Polyurethane, chlorierte Polyether, Casein-Kunststoffe, Phenol-Harz, Harnstoff-Harz, Thioharnstoff-Harz, Melamin-Harz, Epoxidharz, vernetzte Polyurethane, Alkydharz, Allylharz, Silicon, Polyimid, und/oder Polybenzimidazol verwendet werden. Durch die Verwendung von Kunststoff bei der Ausgestaltung des Schnarchtherapiegerätes, kann die Verletzungsgefahr des Benutzers drastisch reduziert werden, da die gummi-elastischen Ausführungsformen keine Oberflächen verletzen. Des Weiteren können durch die Verwendung von Kunststoffen für die Ausgestaltung Kosten reduziert werden.

Es ist bevorzugt, dass das Schnarchtherapiegerät aus einem Polymer, bevorzugt einem Photopolymer, besonders bevorzugt einem Photopolymerharz besteht. Polymere bezeichnen im Sinne der Erfindung insbesondere eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bei solchen sogenannten polymereinheitlichen Stoffen sind also alle Makromoleküle gleich aufgebaut und unterscheiden sich lediglich durch ihre Kettenlänge (Polymerisationsgrad). Man kann derartige Polymere als Polymerhomologe bezeichnen. Polymere können aus der Gruppe umfassend anorganische Polymere, metallorganische Polymere voll- oder teilaromatischen Polymeren, Homopolymere, Copolymere, Biopolymere, chemisch modifizierte Polymere und/oder synthetische Polymere ausgewählt werden und umfassen Polyethylen, Polypropylen, Polyvinylchlorid, Polystyrol, Polymethylmethacrylat, Polyamid, Polyester, Polycarbonat, Polyethylenterephthalat, Polyethylenglykol, Dendrimere, Silikone, Kohlenhydrate oder Polyhydroxxyalkanoate. Das aus diesen Materialien gefertigte Schnarchtherapiegerät stellt einen technischen Fortschritt dar, da durch die bevorzugte Ausführungsform die Verletzungsgefahr reduziert wird und ein Verrutschens des Unterkiefers verhindert wird. Das Material weist feder-elastische Eigenschaften auf und verhindert so eine Verletzung der Oberflächen mit denen das Schnarchtherapiegerät in Kontakt kommt. Photopolymere bezeichnen im Sinne der Erfindung insbesondere Polymere, deren Eigenschaften sich unter Lichteinfluß ändern. In einer bevorzugten Ausführungsform wird Photopolymerharz verwendet. Die Polymere können bevorzugt identische und/oder nicht-identische Härtegrade aufweisen.

Insbesondere werden FullCure-Materialien verwendet, die eine hohe Biegefestigkeit und Wärmeformbeständigkeit aufweisen und für einen breiten Anwendungsbereich mit Anforderungen an Optik, Elastizität, Festigkeit und Belastbarkeit, geeignet sind. Die Materialien sind bevorzugt blickdicht und fest und können für extrem belastbare und formbeständige Modelle mit hervorragender Detailwiedergabe verwendet werden. Es können Modelle erstellt werden, die den umfangreichen Anforderungen an Passgenauigkeit, Form, Funktion und haptischen Eigenschaften genügen. Das bevorzugte Material eignet sich für Modelle mit einer unbegrenzten Bandbreite an komplexen Geometrien, einschließlich Überhänge und Unterschnitte. Bevorzugte Materialien umfassen EC500, HTM140, Iflex500, LS600, R5 Gray, RC31, RC25, E-Dent100, Photosilver, PIC100 Green/Yellow, R5/R11, SI500, WIC100, WIC300, HTM140 HR, EC1000, QID200, WIC402, E-Dent oder HTM140 IV. Besonders bevorzugt sind zudem Materialien umfassend E-Shell200 Series, E-Shell300 Series, E-Shell800, E-Shell600, E-Shell500 Series, E-Shell400 Series oder Kombinationen hieraus. Der angesprochene Fachmann weiß, die genannten Abkürzungen mit Inhalt zu füllen und wie diese Materialien herzustellen bzw. einzukaufen sind. Es war völlig, dass die bevorzugten Materialien, insbesondere Materialien umfassend E-Shell200 Series, E-Shell300 Series, E-Shell800, E-Shell600, E-Shell500 Series, E-Shell400 Series, zur Herstellung eines Schnarchtherapiegerätes eingesetzt werden können. Die Bioverträglichkeit ist gegeben und die Materialien weisen ein geringes Gewicht auf. Somit kann ein Schnarchtherapiegerät hergestellt werden, welches für den Träger angenehm zu tragen ist. Außerdem hat sich in Versuchen herausgestellt, dass die Materialien nicht durch die im Mundraum vorkommenen Enzyme oder den dort herschenden pH-Wert angegriffen werden, so dass eine lange Haltbarkeit garantiert ist. Die im Stand der Technik offenbarten Schnarchtherapiegeräte zeigen zahlreiche bakterielle Verunreinigungen, die eine regelmäßige Desinfektion nach sich ziehen. Hierdurch kann aber wiederum das Material der bekannten Schnarchtherapiegeräte angegriffen werden. Diesbezüglich war es völlig überraschend, dass das bevorzugte Schnarchtherapiegerät mit den bevorzugten Materialien inert gegenüber Mikroorganismen ist, da seine Oberfläche keine Anheftung von Mikroorganismen ermöglicht. Zudem kann durch das bevorzugte Herstellungsverfahren eine im Wesentlichen ebene und glatte Oberfläche bereitgestellt werden, die keine Anheftungsbereiche für Mikroorganismen oder sonstige Verunreinigungen aufweist. Es ist bevorzugt, dass für ein bevorzugtes Schnarchtherapiegerät mehrere, insbesondere unterschiedliche Materialien für die Herstellung der Schienen herangezogen werden. Diesbezüglich kann es besonders bevorzugt sein, dass die Materialien unterschiedliche Shore-Härten aufweisen, so dass ein Schnarchtherapiegerät bereitgestellt wird, welches Bereiche mit unterschiedlicher Shore-Härte besitzt. Das heißt, die Ober- und/oder Unterkieferschiene umfassen in einer bevorzugten Ausführungsform des Schnarchtherapiegerätes Materialien unterschiedlicher Shore-Härten, wobei die Bereiche deutlich voneinander getrennt oder ineinander laufend ausgestaltet sein können.

Die Erfindung wird nunmehr anhand von Figuren beispielhaft beschrieben, ohne jedoch auf diese beschränkt zu sein; es zeigt:
- Fig. 1-4: Modell eines Kiefers und eines Schnarchtherapiegerätes
- Fig. 5-11: Modell eines Schnarchtherapiegerätes
- Fig. 12A-C u. Fig. 13A-C Fig. 15A-E: Bevorzugte Ausführungen eines Schnarchtherapiegerätes
- Fig. 14A-C: Bevorzugte Ausführungsform der Zugbänder

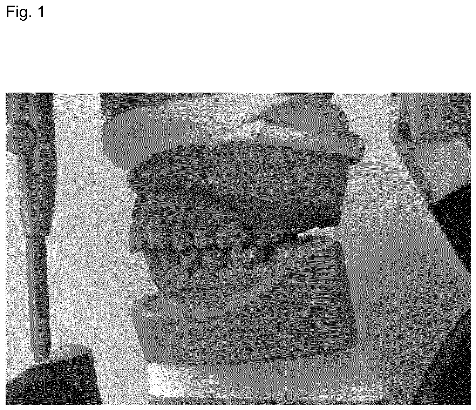

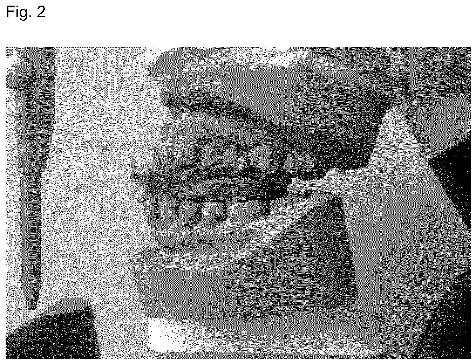

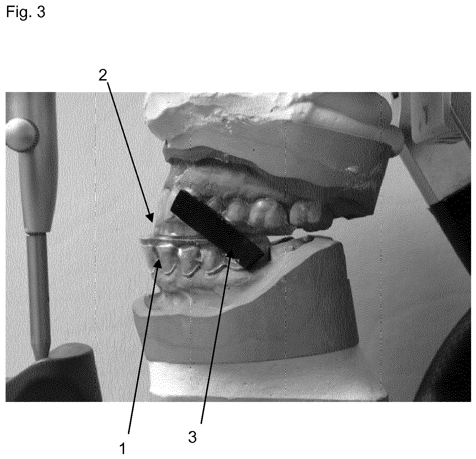

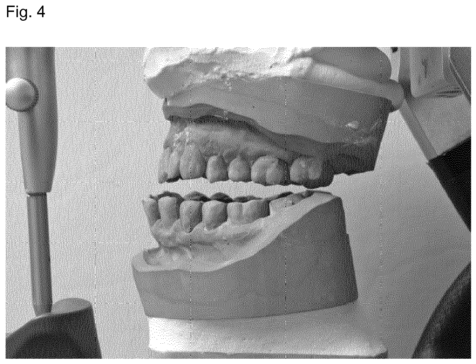

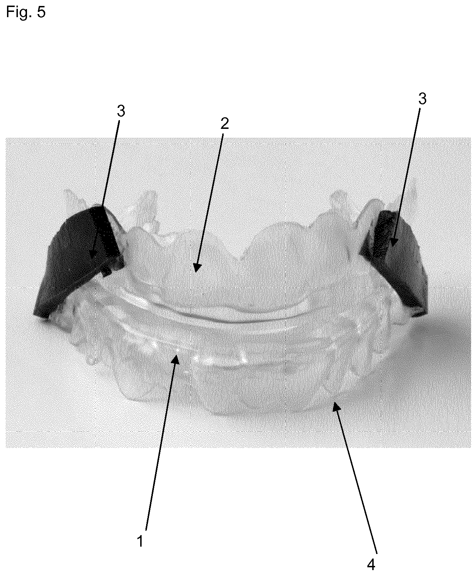

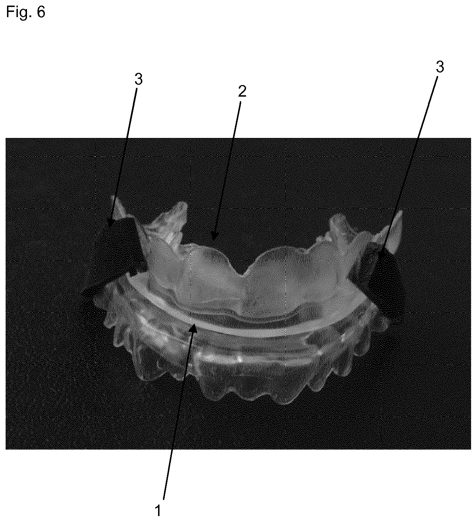

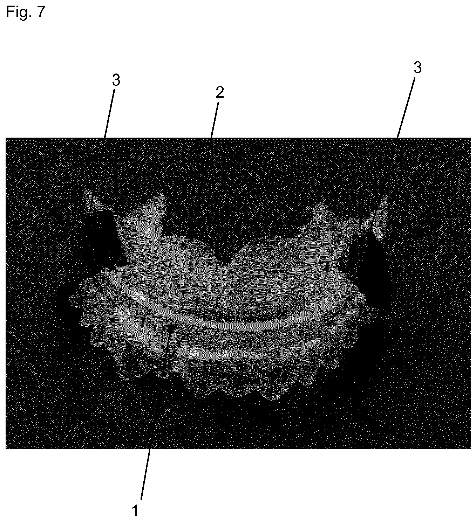

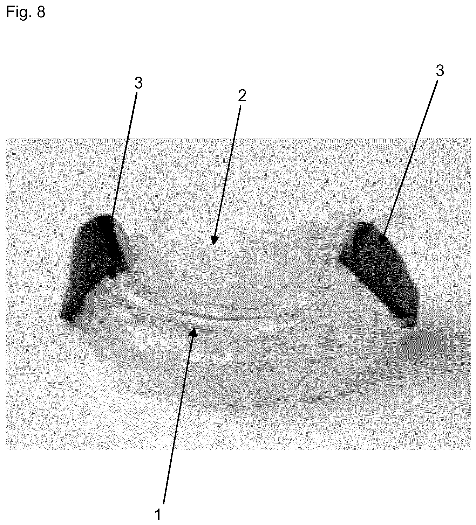

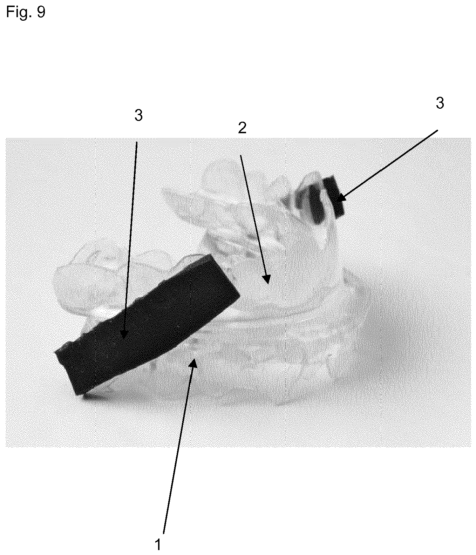

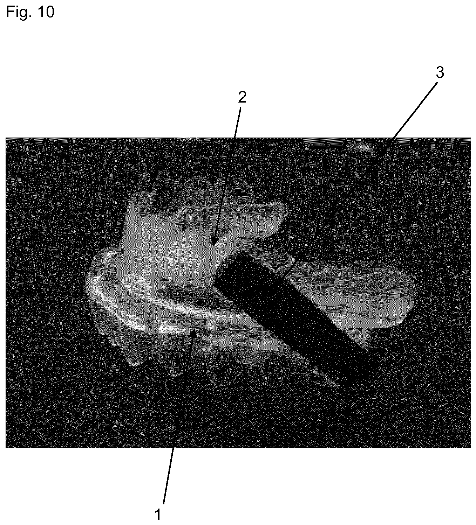

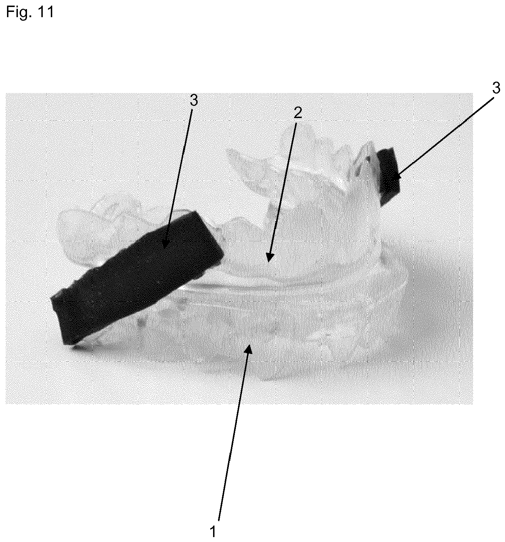

Die Fig. 1 bis Fig. 4 zeigen Modell eines Kiefers und eines Schnarchtherapiegerätes, wohingegen die Fig. 5-11 Modelle eines Schnarchtherapiegerätes in unterschiedlichen Ansichten zeigen. Um ein Schnarchtherapiegerät 4 herzustellen, wird vorzugsweise eine digitale oder analoge Abformung eines Kiefers oder mindestens eines Zahnes vorgenommen und ein Abdruck desselbigen generiert. Der Abdruck kann beispielsweise digitalisiert werden (falls ein analoger Abdruck vorliegt) und mittels einem CAD-System ("Computer Aided Design") bearbeitet werden. Durch das System ist es möglich, das Schnarchtherapiegerät 4 umfassend eine Unterkieferschiene 1, eine Oberkieferschiene 2 und zwei Zugbänder 3 an einem Computer zu gestalten und Schichten mit einem unterschiedlichen Härtegrad zu verwenden. So kann es beispielsweise vorteilhaft sein, dass zwei, drei oder mehr Schichten auf das Modell aufgetragen werden, wobei die Schichten auch aus unterschiedlichen Materialien bestehen können. Vorteilhafterweise können die Härtegrade der Schichten ineinander verlaufen, so dass an definierten Stellen eine bestimmte Härte erreicht werden kann. Beispielsweise kann es bevorzugt sein, dass auf einem Zahn eine erste Schicht vorliegt, die einen geringen Härtegrad aufweist. Eine weitere Schicht kann die zur Mundhöhle hin orientierte Schicht darstellen. Diese Schicht kann beispielsweise einen hohen Härtegrad aufweisen, damit ein ausreichender Schutz vor Verletzung garantiert ist. Es kann jedoch bevorzugt sein, eine Zwischenschicht anzuordnen, um hierdurch die Stabilität des Schnarchtherapiegerätes 4 zu erhöhen. Vorteilhafterweise können die Schichte unterschiedliche Härtegrade aufweisen und bevorzugt aus unterschiedlichen Materialien bestehen, wobei die Härtegrade ineinander verlaufen. Dies ist mit dem im Stand der Technik beschriebenen Folientiefziehverfahren, bei dem die Folien thermoplastisch miteinander verbunden werden, nicht möglich. Es kann so ein Schnarchtherapiegerät 4 bereitgestellt werden, das eine optimale Passung und Stabilität aufweist. Die Fertigung des Schnarchtherapiegerätes erfolgt vorteilhafterweise mittels einem CAM-System ("Computer Aided Modulation"). Bevorzugt ist ein 3D-Drucker, der eine schnelle und präzise Fertigung des Schnarchtherapiegerätes 4 garantiert. Durch das bevorzugte Verfahren kann schnell und einfach Schnarchtherapiegerät 4 bereitgestellt werden, das in wenigen Arbeitsschritten herstellbar ist und mittels einem CAD/CAM-System kreiert und fertiggestellt wird. Fig. 1 zeigt die Stellung des Kiefers vor der Anwendung mit dem Schnarchtherapiegerät 4 und Fig. 4 die Stellung des Unterkiefers während der Behandlung mit dem Gerät 4.

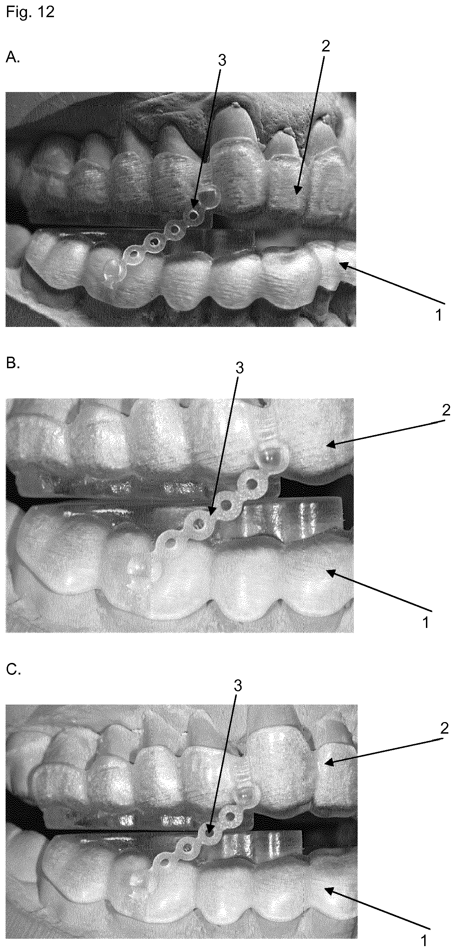

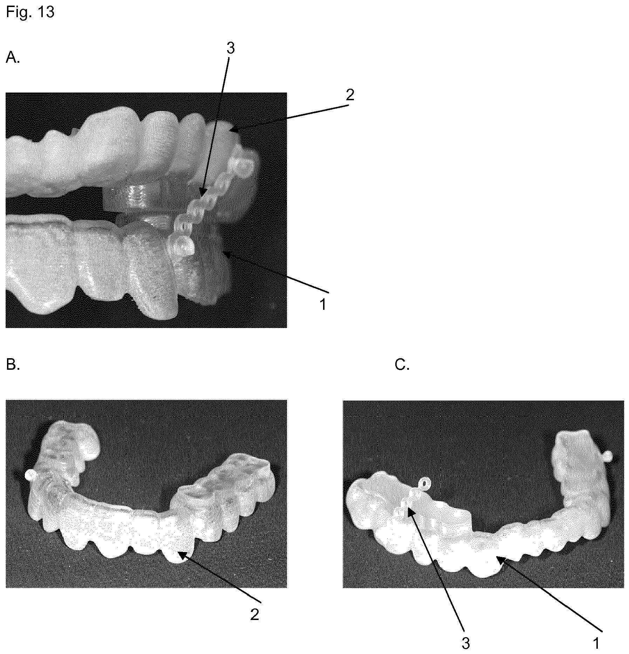

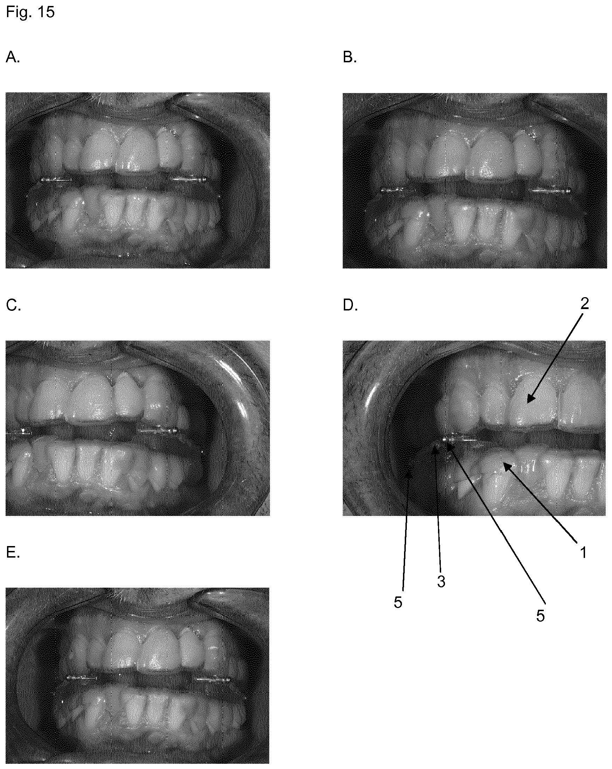

Fig. 12A-C, Fig. 13A-C und Fig. 15A-E zeigen bevorzugte Ausführungen eines Schnarchtherapiegerätes. Das Zugband 3 weist vorzugsweise Einstellmöglichkeiten auf, die eine individuelle Anpassung der Schienen 1, 2 zueinander ermöglichen. Hierdurch kann die Lage der Oberkieferschiene2 zur Unterkieferschiene 1 verschoben bzw. eingestellt werden. Das Zugband 3 kann insbesondere so ausgestaltet sein, dass es in Befestigungsmittel an den Schienen angebracht wird. Eine einfache Ausgestaltung des Befestigungsmittels kann beispielsweise eine Ausstülpung sein, wobei das Zugband 3 Durchstöße (Löcher) aufweist, in die die Ausstülpungen eingebracht werden. Die Ausstülpungen, die vorzugsweise an den Seiten der Schienen 1, 2 angeordnet sind und als Zugbandhalterungen bezeichnet werden können, liegen vorzugsweise nicht in einer vertikalen Ebene zueinander, sondern schräg (insbesondere in einem Winkel von 20° bis 60°, vorzugsweise 40°). Die Ausstülpungen können aus einem Kunststoff oder Metall gefertig sein. Die Zugbänder 3, die im Sinne der Erfindung auch als Kunststoffketten oder Alastikketten bezeichnet werden, können in verschiedenen Farben und Größen hergestellt werden. Außerdem können sie mit oder ohne einem Steg ausgestaltet sein. Bevorzugte Materialien umfassen thermoplastische Materialien, insbesondere Polyurethan, Polyester, Elastomer, Polyurethan aus Methylendiphenyl Diisocyanat, 1, 4-Butandiol, Polytetramethylende Glykol oder Kombinationen hieraus. Es kann auch bevorzugt sein, wenn die Ausstülpungen 5 als Kugelknöpfe aus Metall in die geplotteten Schienen 1, 2 eingearbeitet werden, wobei es auch bevorzugt sein kann, wenn die Kugelknöpfe nachträglich eingebracht werden. Die Kugelköpfe dienen insbesondere der Anbringung, bzw. Befestigung des Zugbandes 3. Das heißt, die Ausstülpungen können bevorzugt aus Metal oder Kunststoff gefertigt sein.

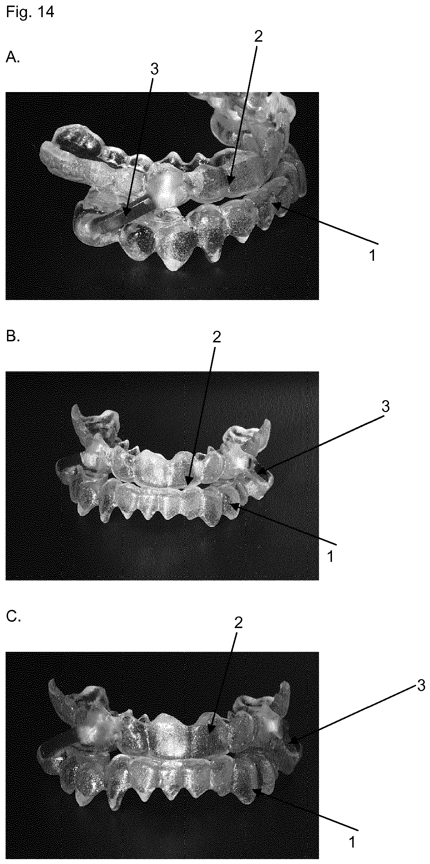

Fig. 14 A-C zeigte eine bevorzugte Ausführungsform der Zugbänder. Es kann auch bevorzugt sein, dass das Zugband 3 keine Ausstülpungen oder Durchstöße aufweist und in einem Schritt mit der Unterkiefer- und Oberkieferschiene 1,2 geplottet wird und somit mit den Schienen 1, 2 fest verbunden ist. Hierbei ist das Zugband 3 fest, bevorzugt irreversibel mit den Schienen 1, 2 verbunden, wobei das Schnarchtherapiegerät einfach, schnell und kostengünstig spezifisch für einen Patienten herstellbar ist. Vorzugweise können die Schienen 1, 2 bzw. das Zugband 3 aus unterschiedlichen Materialien hergestellt werden, insbesondere ein transparentes Material: Full Cure 720 und ein schwarzes Material Tango Black. Das Zugband 3, bzw. die beiden Zugbänder 3 können elastisch ausgestaltet sein, wobei es auch bevorzugt sein kann, dass sie keine Elastizität aufweisen.

### Bezugszeichenliste

- 1: Unterkieferschiene
- 2: Oberkieferschiene
- 3: Zugband
- 4: Schnarchtherapiegerät
- 5: Ausstülpungen

## Patentansprüche

1. Intraorales Schnarchtherapiegerät mit einer Oberkieferschiene und einer Unterkieferschiene, wobei die Oberkieferschiene und die Unterkieferschiene über mindestens zwei Zugbänder miteinander verbunden sind und die Zugbänder mit ihrem ersten Ende in einer Ausnehmung im seitlichen Bereich der Oberkieferschiene und ihrem zweiten Ende in einer Ausnehmung im seitlichen Bereich der Unterkieferschiene befestigt sind, so dass die Oberkieferschiene und die Unterkieferschiene relativ zueinander bewegbar sind, wobei die Zugbänder eine Shore-Härte von 20 bis 70 haben und die Zugbänder derart an der Oberkieferschiene und an der Unterkieferschiene befestigt sind, dass der Unterkiefer im Gebrauchszustand bei in den Mund eingesetztem Therapiegerät mit dem Öffnen des Mundes nach vorne bewegt wird, wobei die Oberkieferschiene und/oder die Unterkieferschiene eine Dicke von 3 - 6 mm aufweist.

2. Schnarchtherapiegerät nach Anspruch 1, **dadurch gekennzeichnet, dass**
die Oberkieferschiene und die Unterkieferschiene mindestens teilweise ebene Flächen aufweisen, welche sich bei in den Mund eingesetztem Therapiegerät im Bereich der Kauflächen auf den sich zugewandten Seiten der Schienen befinden.

3. Verfahren zur Herstellung eines Schnarchtherapiegeräts nach Anspruch 1 oder 2, umfassend die folgenden Schritte:
a. analoge und/oder digitale Abformung mindestens eines Kiefers oder eines Zahnes,
b. Datenaufbereitung der unter a. bereitgestellten Abformung,
c. digitale Modulation des Schnarchtherapiegeräts und
d. Fertigung des Schnarchtherapiegeräts.

4. Verfahren nach Anspruch 3, **dadurch gekennzeichnet, dass**
die analoge Abformung Abdruckverfahren umfasst.

5. Verfahren nach Anspruch 3 oder 4,
**dadurch gekennzeichnet, dass**
die digitale Abformung optisch erfolgt.

6. Verfahren nach einem oder mehreren der vorherigen Ansprüche 3-5,
**dadurch gekennzeichnet, dass**
die Datenaufbereitung scannen, digitalisieren, speichern und/oder umwandeln umfasst.

7. Verfahren nach einem oder mehreren der vorherigen Ansprüche 3-6,
**dadurch gekennzeichnet, dass**
das Schnarchtherapiegerät mit einem 3D-Drucker, insbesondere einem Multilayerdrucker gefertigt wird
und/oder
das Schnarchtherapiegerät aus einem Polymer, bevorzugt einem Photopolymer, besonders bevorzugt einem Photopolymerharz besteht.

8. Verfahren nach einem oder mehreren der vorherigen Ansprüche 3-7,
**dadurch gekennzeichnet, dass**
das Schnarchtherapiegerät aus mehreren homogen miteinander verbundenen Polymerlagen besteht.

9. Verfahren nach Anspruch 8 **dadurch gekennzeichnet, dass**
die Polymerlagen identische und/oder nicht-identische Härtegrade aufweisen.

10. Schnarchtherapiegerät nach einem der Ansprüche 1 - 2 **dadurch gekennzeichnet, dass** das Schnarchtherapiegerät in Übereinstimmung mit einem Zahnbogen ausgeformt ist.

## Claims

1. An intraoral snoring therapy device having a maxillary splint and a mandibular splint, wherein said maxillary splint and said mandibular splint are interconnected by at least two straps and said straps are attached with their first end in a recess in the lateral region of the maxillary splint and with their second end in a recess in the lateral region of the mandibular splint in such a way that said maxillary splint and said mandibular splint are movable relative to one another, wherein said straps have a Shore hardness of 20 to 70 and said straps are attached to said maxillary splint and said mandibular splint in such a way that, with said therapy device in the use position inserted in the mouth, the mandible is moved forward with the opening of the mouth, wherein the maxillary splint and/or the mandibular splint has a thickness of 3 to 6 mm.

2. The snoring therapy device according to claim 1, **characterized in that**
said maxillary splint and said mandibular splint have at least partially flat surfaces which are located in the region of the occlusal surfaces on the sides of the splints facing one another with said therapeutic device inserted in the mouth.

3. A method of producing a snoring therapy device according to claim 1 or 2, comprising the following steps:
a. analog and/or digital impression of at least one jaw or tooth,
b. data preparation of the impression prepared in a.,
c. digital modulation of said snoring therapy device, and
d. production of said snoring therapy device.

4. The method of claim 3,
**characterized in that**
said analog impression comprises impression methods.

5. The method according to claim 3 or 4,
**characterized in that**
said digital impression takes place optically.

6. The method according to one or more of the preceding claims 3-5,
**characterized in that**
said data preparation comprises scanning, digitizing, storing, and/or converting.

7. The method according to one or more of the preceding claims 3-6,
**characterized in that**
said snoring therapy device is manufactured with a 3D printer, in particular a multilayer printer
and/or
said snoring therapy device is made of a polymer, preferably a photopolymer, particularly preferably a photopolymer resin.

8. The method according to one or more of the preceding claims 3-7,
**characterized in that**
the snoring therapy device is made of several homogeneously interconnected polymer layers.

9. The method according to claim 8
**characterized in that**
said polymer layers have identical and/or non-identical degrees of hardness.

10. The snoring therapy device according to any one of the claims 1-2,
**characterized in that** the snoring therapy device is formed in accordance with a dental arch.

## Revendications

1. Dispositif intrabuccal de traitement du ronflement avec une attelle de mâchoire supérieure et une attelle de mâchoire inférieure, dans lequel l'attelle de mâchoire supérieure et l'attelle de mâchoire inférieure sont reliées l'une à l'autre via au moins deux tirants et les tirants sont fixés par leur première extrémité dans une cavité de la zone latérale de l'attelle de mâchoire supérieure et par leur seconde extrémité dans une cavité de la zone latérale de l'attelle de mâchoire inférieure, de sorte que l'attelle de la mâchoire supérieure et l'attelle de la mâchoire inférieure puissent se déplacer l'une par rapport à l'autre, dans lequel les tirants ont une dureté Shore de 20 à 70 et les tirants sont fixés à l'attelle de mâchoire supérieure et à l'attelle de mâchoire inférieure en sorte que la mâchoire inférieure soit déplacée vers l'avant en état de service dans le dispositif de traitement utilisé dans la bouche avec l'ouverture de la bouche, dans lequel l'attelle de mâchoire supérieure et/ou l'attelle de mâchoire inférieure présente(nt) une épaisseur de 3 à 6 mm.

2. Dispositif de traitement du ronflement selon la revendication 1,
**caractérisé en ce que**
l'attelle de mâchoire supérieure et l'attelle de mâchoire inférieure présentent au moins en partie des surfaces planes, qui se trouvent, dans le dispositif de traitement utilisé dans la bouche, dans la zone des surfaces de mastication sur les côtés des attelles tournés vers celles-ci.

3. Procédé de fabrication d'un dispositif de traitement du ronflement selon la revendication 1 ou 2, comprenant les étapes suivantes :
a. moulage analogique et/ou numérique d'au moins une mâchoire ou une dent,
b. traitement de données du moulage effectué sous a.,
c. modulation numérique du dispositif de traitement du ronflement et
d. production du dispositif de traitement du ronflement.

4. Procédé selon la revendication 3,
**caractérisé en ce que**
le moulage analogique comprend un procédé de reproduction.

5. Procédé selon la revendication 3 ou 4,
**caractérisé en ce que**
le moulage numérique se fait en mode optique.

6. Procédé selon une ou plusieurs des revendications précédentes 3 à 5,
**caractérisé en ce que**
le traitement de données comprend un balayage, une numérisation, une mémorisation et/ou une reproduction.

7. Procédé selon une ou plusieurs des revendications précédentes 3 à 6,
**caractérisé en ce que**
le dispositif de traitement du ronflement est reproduit avec une imprimante 3D, en particulier une imprimante multicouche, et/ou
le dispositif de traitement du ronflement est constitué d'un polymère, de préférence d'un photopolymère, tout particulièrement d'une résine de photopolymère.

8. Procédé selon une ou plusieurs des revendications précédentes 3 à 7,
**caractérisé en ce que**
le dispositif de traitement du ronflement est constitué de plusieurs couches de polymère reliées l'une à l'autre de manière homogène.

9. Procédé selon la revendication 8,
**caractérisé en ce que**
les couches de polymère présentent des degrés de dureté identiques et/ou non identiques.

10. Dispositif de traitement du ronflement selon l'une quelconque des revendications 1 à 2, **caractérisé en ce que** le dispositif de traitement du ronflement est formé en conformité avec une arcade dentaire.
